# EUROPEAN PATENT APPLICATION

(11) **EP 3 399 449 A1**
(43) Date of publication of application: **07.11.2018**
(21) Application number: 17169080.3
(22) Date of filing: 02.05.2017
(51) Int. Cl.: G06F 19/00

(54) **DIAGNOSTIC SUPPORT IN AN X-RAY SYSTEM**

(71) Applicant: Koninklijke Philips N.V., 5656 AE Eindhoven (NL)
(72) Inventor: VOGTMEIER, Gereon, 5656 AE Eindhoven (NL); YOUNG, Stewart, 5656 AE Eindhoven (NL); CHAKRABARTI, Biswaroop, 5656 AE Eindhoven (NL); RAGHOTHAM VENKAT, Prasad, 5656 AE Eindhoven (NL)
(74) Representative: de Haan, Poul Erik

(57) **Abstract**

The present invention relates to an X-ray device, an X-ray system and the method of operation thereof. Support by remote image-analysis expertise is performed automatically by executing a decision escalation chain.

## Description

### FIELD OF THE INVENTION

The present invention relates to X-ray systems and devices and the operation thereof.

### BACKGROUND OF THE INVENTION

X-ray systems can potentially become an attractive technology, for example in locations at large distances from institutional healthcare facilities or other professional medical infrastructure. Such systems may be operated by personnel with a lower level of formal training than typically provided in healthcare institutions.

### SUMMARY OF THE INVENTION

There may thus be a need to provide a tailored support service which is appropriate for the needs of the specific usage situation of a X-ray system or X-ray device.

According to at least some examples, the features provided by the invention as described in the following are also particularly for a mobile X-ray device, a mobile X-ray system and a method of operation thereof. A mobile X-ray device, when used in rural areas or nursing homes, is brought to the patient for scanning procedures. Furthermore, at least some features of the invention presented herein also apply to other mobile X-ray health systems.

The object of the present invention is solved by the subject-matter of the independent claims; further embodiments are incorporated in the dependent claims. It should be noted that the following described aspects of the invention apply also for the X-ray device, the X-ray system as well as for the described method for image acquisition by use of the X-ray system and vice versa. According to at least some examples, the features provided by the invention as described in the following are also particularly for a mobile X-ray device, a mobile X-ray system and a method of operation thereof. A mobile X-ray device, when used in rural areas or nursing homes, is brought to the patient for scanning procedures. Furthermore, at least some features of the invention presented herein also apply to other mobile X-ray health systems.

According to the present invention an X-ray device and system and a method for operation thereof is provided, wherein remote image-analysis expertise is automatically requested according to an agreement level of an automatic analysis of an X-ray image and an operator's interpretation thereof. The remote image-analysis expertise request is performed automatically by performing a decision escalation chain.

According to the present invention an X-ray device is provided comprising an X-ray source, a detector, a processing unit and an interface for receiving the operator decision of the X-ray image and for requesting remote image-analysis expertise according to one of the agreement level, operator decision and/or automatic analysis result. The request for remote image-analysis expertise is performed automatically by executing a decision escalation chain. The processing unit comprises image acquisition for generating an X-ray image, performing automatic image analysis of the X-ray image and comparing an automatic analysis result and an operator decision for the X-ray image and monitoring an agreement level of the comparison.

When an X-ray device is operated by personnel (operator) with a lower level of formal training than that typically provided in healthcare institutions, it could be necessary to provide enhanced levels of automated support (with respect to current systems) in order to enable a required flexibility in deployment of such systems. The level of automation needed to support the operator will depend upon a range of factors, including the qualification of the operator, diagnostic demands or requirements for the imaged object (the object is a patient, for example, with a healthcare insurance and/or a treatment history), and the context of the situation in which the imaging is performed (e.g. emergency, elderly-care). Besides operation of the X-ray system, a further aspect for safe usage of such systems is the workflow of the associated (diagnostic) decision following image acquisition. Provision of remote image-analysis support can enhance the reliability of diagnoses for the imaged patient. Remote image-analysis support is available by second-reader diagnoses of selected experts connected to the X-ray system via a communication network.

According to the present invention, also an X-ray system is provided, comprising an X-ray device and a connection to a remote expertise system. The request for remote image-analysis expertise is performed automatically by executing a decision escalation chain.

According to the present invention, a method for image acquisition by use of an X-ray system is provided. The X-ray system comprises an X-ray device. The method comprising the following steps: generating an X-ray image; performing automatic image analysis of the X-ray image; receiving an operator decision of the X-ray image; monitoring an agreement level of an automatic analysis result and the operator decision; requesting remote image-analysis expertise according to one of the agreement level, operator decision and/or automatic analysis result. According to the method, the requesting of remote image-analysis expertise is performed automatically by executing a decision escalation chain.

In order to identify an appropriate level of support for analyzing a generated X-ray image, in some examples, the requested level of support is performed automatically by executing a decision escalation chain according to the operator expertise level. The operator expertise level is identified according to the provided level of training and/or professional qualification which is known to the X-ray system by provision of an according database, for example.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 an X-ray system according to the invention;
Fig. 2 a method for image acquisition according to the invention;

### DETAILED DESCRIPTION OF EMBODIMENTS

According to the invention, image acquisition by use of an X-ray system is performed. The generated X-ray image is subject to automatic image analysis. The generated X-ray image is analyzed by an operator of the X-ray device. The operator decision of the X-ray image and the result of the automatic analysis are monitored. According to the agreement level of the operator decision and the automatic analysis result remote image-analysis expertise is requested. Also, if operator decision and automatic analysis result agree, but one or both results are less reliable remote image-analysis expertise is requested. The request is performed automatically by executing a decision escalation chain.

According to the present invention, the monitoring of an agreement level between operator decisions and the provided results of the automatic image analysis system ensures safe diagnosis results. Agreement between these decisions leads to a pre-defined next step for the patient treatment; disagreement leads automatically to the next escalation level, executed by the decision escalation chain. In the next escalation level additional remote image-analysis expertise is consulted.

The agreement level, in some examples, is based on several criteria. Such criteria are, e.g. the expected diagnosis matches the provided diagnosis; image information processing and interpretation leads to the same diagnosis; quantitative image data like contrast provided by the generated image is analyzed in the same way; matching of diagnosis according to a patient history and the like.

As a default, the request for remote image-analysis expertise is performed automatically by executing a decision escalation chain. In some examples, the decision escalation chain is provided in the X-ray device. In some examples, the decision escalation chain is configured to be executed automatically for specific medical fields, while other medical fields are subject to manual request for remote image-analysis expertise. Such configuration, automatic or manual, is provided by the X-ray system. In some examples, e.g. in a hospital, remote image-analysis expertise could be provided by an expert on site, so that automatic execution of the decision escalation chain means confirmation/rejection of a diagnose by a physician or other highly qualified expert.

According to an example, the best matching remote image-analysis expertise is provided when executing the decision escalation chain. The decision escalation chain is executed according to information about the condition of the patient (suspected diseases, conditions), any available prior knowledge of the patient which could be made available to the system.

According to some examples, the automatic execution of the decision escalation chain for establishing the highest level of confidence in the resulting diagnosis is implemented automatically in the X-ray device. Guidance along a step-by-step decision menu can be provided and the individual steps can be logged or stored to establish a documentation of the decision-making process - also in case an operator provides incorrect input information to the X-ray system.

According to an example, diagnostic demands and/or requirements are determined prior to image acquisition. Based thereon, possible outcomes of patient diagnosis can be estimated, yielding consequences (i) for the operation of the system, and (ii) for the diagnosis and the treatment decision provided by the operator. Diagnostic demands and/or requirements comprise patient's wishes which could include personal references but also financial arguments (insurance, extra payment, etc.), request for specific remote image-analysis expertise, which could be a particular physician consulted by the patient before, a dedicated specialized expert and the like.

According to an example, the operator simply operates image acquisition, reviews the acquired image and proposes an operator decision of the X-ray image or further course of action, without a need of remote image-analysis expertise. For example, if a bone is broken and this is clearly visible in the radiograph (generated X-ray image), immediate action can be taken. In a more complicated case, the operator decision of the X-ray image and the automatic analysis result provided by the automatic image analysis system might result in a low level agreement.

According to an example, if the operator's decision of the X-ray image and the automatic analysis result concur, the agreement level is classified as high, based on the assumption that there is a high probability that the operator decision is an accurate result. For a high agreement level, at least both (automatic analysis result and operator decision) confirm with high degree of confidence a possible diagnosis. Classification of the agreement level can be configured according to the medical field, number of previous similar analyzed images and provided diagnosis, treatment or further action. Thus, in some medical fields an agreement level might be classified as high only if additional criteria such as a high confidence level of the automatic analysis is provided. In some examples, the level of agreement might depend on the operator expertise level.

Each generated X-ray image and the according method of operation including diagnosis, treatment, request for remote image-analysis expertise, is recorded and stored for follow-up. Such data can be analyzed and provided as automatic analysis results for future operation of the X-ray system. Accordingly, automatic analysis results can be provided with confidence levels. The level of confidence could be based, e.g. on the number of stored cases where the same diagnose, further action or support is given for similar generated X-ray images. This leads to highly reliable automatic analysis results provided by the system and increases the reliability of the method of operation, which in some examples could overrule the operator decision.

According to an example, if the agreement level of the comparison is low or if the operator decision and/or the automatic analysis result provided by the image analysis system is classified as having low quality or low confidence level or if these results conflict, then remote image-analysis expertise is requested, e.g. a second reader confirmation, expert support or comparison with similar images of the patient provided by a database.

Image analysis system performs image processing on site and image understanding (e.g. Computer Aided Diagnosis) system. The comparison is performed by a processing unit of the X-ray device of the X-ray system. In some examples, the image analysis system uses deep learning algorithm by using the performed and provided images along with the provided diagnoses, treatments, further course of action. Deep learning can be performed by extraction of the most utilized results, which are provided with confidence levels according to quality criteria, number of stored images and the like. Also, big data analysis methods could be utilized, e.g. to find correlation values between the generated X-ray images and the resulting diagnosis, treatment or further course of action. Accordingly, the decision escalation chain can be determined.

According to the present invention, an X-ray system is provided for optimizing the support for an operator of the X-ray imaging system. At least two aspects of the provided X-ray system are: (i) handling of the workflow on site up to a decision point where remote image-analysis expertise is deemed as necessary or the analysis could be performed by the operator on site, and (ii) if remote image-analysis expertise is requested, the selection of the best matching remote image-analysis expertise.

According to some examples, the operation of the X-ray system corresponds to the expertise level of the operator. Accordingly, the X-ray device comprises an interface for adaptive interaction of an operator and the X-ray device. Such adaptive interaction of the X-ray system with the operator ensures good operation quality and operator guidance whenever necessary. In addition to identification data of the operator also information about a training level of the operator is provided.

According to an example, the method of operation comprises determining an operator expertise level. The processor unit comprises determining the operator expertise level. In some examples, ID-means are provided for identification of the operator and/or object.

According to an example, the level of interaction and/or operation is provided by at least one option selector according to the operator expertise level and is outputted by the interface. The outputted option selector can thus provide more freedom of choice for an operator expert to select protocols, for example, while a non-expert operator could perform the same or a similar way of operation only by following a step-by-step guidance provided by the at least one option selector. Protocols are scanning protocols: pediatric, cardiac, small region of interest. Generally, such protocols comprise predefined parameter settings according to a disease or region. Also, the current and voltage range of the X-ray device can be adapted, e.g. range of mA or kV, respectively (e.g. an adult's protocol with high mA is not used for a child, to prohibit overdosing).

In an example, at least one subsequent option selector is provided according to the operator expertise level. The subsequent option selector is adapted to the operator expertise level. This provides a fast way of operation, if the operator expertise level is appropriate as the (subsequent) option selector matches operator's qualification and training.

The at least one or subsequent option selector can also be adapted or modified according to the identified object data. The outputted option selector is selectable via the interface for adaptive interaction of the operator and the X-ray device. The interface for adaptive interaction comprises at least one of a display, a touch-screen, keyboard and the like for entering data and for displaying information.

According to an example, the X-ray system comprises a connection to a remote expertise system which is a brokering system. An information broker could control the subsequent steps of the decision escalation chain and organize the subsequent workflow to ensure the shortest possible time to establish a diagnosis according to the generated X-ray image with high degree of confidence. As a default, the decision escalation chain is performed automatically. Depending on patient and/or operator wishes/requests some selections in the decision escalation chain could be made automatically or manually (e.g. if patient wishes are available for a particular application, disease, system. However, the X-ray system is configurable for selection options. The complete operation and the decision escalation chain is documented and an in some embodiments also depend on the provided financial provisions of a patient, if any

According to some examples remote image-analysis services are organized using a brokering system: according to the determined diagnostic demands and/or requirements different support services are selected following the decision escalation chain. Support services could be second reader, expert consultancy, involvement of physicians who know the patient, patient wish regarding an expert in a hospital that will do treatment, fast results of diagnosis, insurance linked experts and other options.

According to some examples, the brokerage service for image data analysis will also distribute incoming data to the reader stations according to priority request (depending on urgency for the patient), depending on availability or need for in time diagnose as well as financial issues and quality control.

According to some examples, the brokering service assumes responsibility for the integrity and quality (time-wise, certification of the qualification of those involved, data protection, etc. etc.) of the decision-making process. The integrity of the diagnosis decisions remains in the medical responsibility of the analyzing/interpreting operators or physician(s) on site when operating the X-ray system. However, responsibility might change if the confidence level of the system is high enough (i.e. the reliability of the system is confirmed and the system is configured accordingly).

A remote image-analysis expertise could provide solutions for operators of the X-ray system under such circumstances where image quality might be influenced by less adequate arrangement of the X-ray device or less ideal protocol/setting selections. In some examples, such support can be provided by experts who have seen many such images and have gained expertise how to overcome such issues, so that additional information can be provided in order to find the correct diagnosis.

Further involvement of dedicated personnel as second reader include the personal preference of the patient, or prior patient-physician relationship e.g. due to former clinical treatments. It is a demand to establish and maintain the patient trust in the 2nd reader / 2nd reader organization to ensure that the patient accepts the result. Trusted organizations or expert networks might be part of the reader network, comprising specific expertise or experts requested by the patient or insurance pre-defined expertise for the remote diagnosis. Such patient concerns could be managed by a trusted brokering service. Ensuring confidence in the result is an issue. This can be provided as double check of previously obtained patient data and/or additional sensor data and information could be included.

Secure transfer of the data to trusted devices via reliable information channel to protect patient's data privacy should be ensured. In some examples, this is provided for the image data generated by the X-ray system as well as for the additional patient data and the diagnostic results. The reader network comprising expert network, evaluators, specifically qualified expert etc., also has to fulfil the data privacy requirements. Any interaction and data logging should be done accordingly.

According to the present invention an X-ray system is provided as decision escalation chain, so that the workflow and dataflow supports the selection of second-reader diagnoses. Also, a selection of an appropriate partner-service via an image-interpretation brokering service is provided along with automated decision-making process embedded within the X-ray system or X-ray device itself. The provided double checking by the remote image-analysis expertise and comparison with automatic image processing increases and verifies the confidence level of each image acquisition procedure.

According to some examples comparison with automatic generated data could be performed for each step of the decision escalation chain to double check for mistakes or inconsistencies.

According to some examples an overruling function is provided for each step of the decision escalation chain. Such emergency provision is implemented to enable operators to over-rule any automated error-checking performed by the system. However, any such overruling would be logged for subsequent review by other expert operators.

As patients could provide diagnostics demands such as extra services, which influence the decision escalation chain, billing of such extra services as well as the double check for misuse due to unneeded multiple usage can also be provided. Double check of usage of trusted devices like mobile image display devices has to be done due to certified test methods and has to be stored for the individual use cases: documentation of decision flow.

The invention could be used in mobile healthcare systems like X-ray systems with remote image-analysis expertise and diagnose activities that have to be organized. Combining data from several systems, such as operator and image-analysis expertise certainly increases trust in X-ray systems and devices as well as the qualification of the provided methods of operation.

The features of the invention are described using an X-ray system. It should be noted that the following description of the figures is also for a mobile X-ray device, mobile X-ray system or for other mobile health systems or devices.

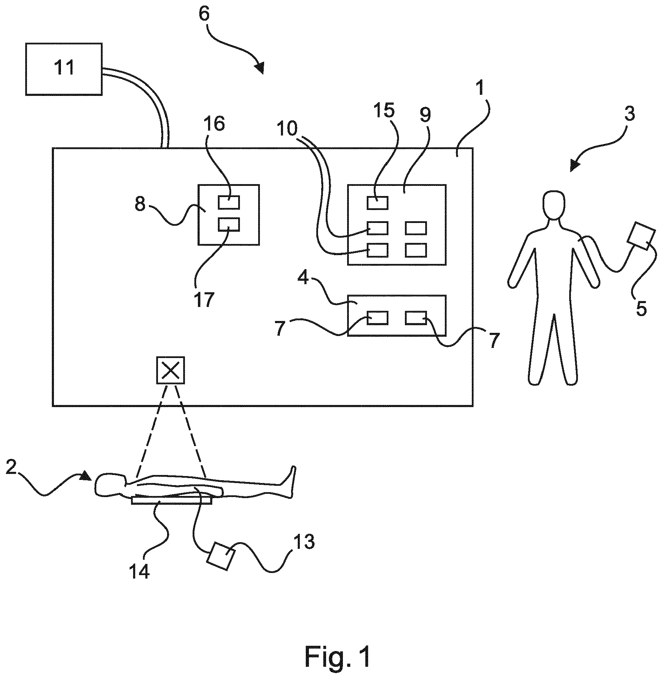

Fig. 1 shows an X-ray system 6 comprising an X-ray device 1. The X-ray device comprises an X-ray source X and a detector 14. An object (patient) 2 is arranged between an X-ray source and a detector 14.

In some embodiments, the X-ray device 1 can also comprise an ID-system 4 with at least one ID-means 7 for identification of an operator 3 and the object 2. The operator 3 is identified via an operator identification device 5 and the object 2 is identified via an object identification device 13. More than one ID-means 7 can be provided to reflect the multiple ways of identification devices which can be detected by the ID-system 4. The operator identification device 5 could have an RFID chip and the identification is performed via a RF link to the ID-system 4 of the X-ray device 1. The identification device 13 of the patient (object 2) can be a mobile phone. The identification can be performed via the phone number, IMEI or finger-print identification provided by the mobile phone. Typical identification devices suitable for identification of the operator 3 or patient (object) 2 are mobile phone, e-health card, ID card with RF link, electronically readable passport. The ID-system 4 and the ID-means 7 are adaptable to any available technology for identification via interaction with the X-ray system 6.

The X-ray device 1 comprises a processor unit 8. The processor unit 8 comprises image acquisition 16 for generating an X-ray image, performs automatic image analysis of the X-ray image and compares an automatic analysis result 17 and an operator decision 15 for the X-ray image and monitors an agreement level of the comparison.

The X-ray device 1 further comprises an interface 10 for receiving an operator decision 15 of the X-ray image and for requesting remote image-analysis expertise provided via a connection to a remote expertise system 11. The request for remote support is performed according to one of the agreement level, operator decision 15 and/or automatic analysis result 17. The request for remote image-analysis expertise is performed automatically by executing a decision escalation chain, as is explained in more detail in the following figures.

In some embodiments, the processor unit 8 is also configured to determine an operator expertise level as expert level E or basic level B by matching the identification information received from the ID-system 4 and the information provided by a database of the remote expertise system 11. In some embodiments, the operator expertise levels can be configured including operator expertise level for specific applications, so that the operator expertise level can be changed during an operation if such specific applications are to be used. Also, the operator expertise level can be configured to include identification of having no expertise in some fields of operation.

In some embodiments, an operator 3 may be admitted registering a new operator via the ID-system 4. In such embodiments, the ID-system 4, the processor unit 8 and/or an interface 9 is configured to perform such registration by providing according output and receiving according input.

The processor unit 8 checks whether the operator 3 is known to the X-ray system 6 as provided in the database of the remote expertise system 11. The database could also be provided or otherwise integrated in the X-ray device 1. The processor unit 8 also checks the patient identification if available in the database 11. If available the patient 2 treatment history can be provided to the processor unit 8 via the database 11.

Accordingly, an operator expertise level is determined and the interface 9 for adaptive interaction of the operator 3 and the X-ray system 1 is provided. The interface 9 is configured to output at least one option selector 10, however the number of option selector 10 is adapted according to the required guidance of the operator 3 depending on the identified operator expertise level. Via the interface 9 the operator 3 can select or even waive the outputted option selector 10, which will be explained in more detail with reference to the other figures.

The operator 3 performs image acquisition by activating the X-ray source X of the X-ray device 1 after all required steps are performed/confirmed by interaction with the X-ray device 1 via the interface 9. The option selector 10 of the interface 9 are adapted, so that an operator 3 can perform image acquisition adapted to its expertise level. This provides a highly flexible X-ray system 6 which can be used in cases such as in a rural kiosk, in a nursing home or in a hospital or other location with multiple users (professional care). If the "expert level database" is available via a mobile phone app or identification link the provided X-ray system could be used also in scenarios for identifying or checking a physician's expertise level in a remote location such as a plane for example, in case there is doubt about the expertise of such person to ensure that only trained personnel treats patients.

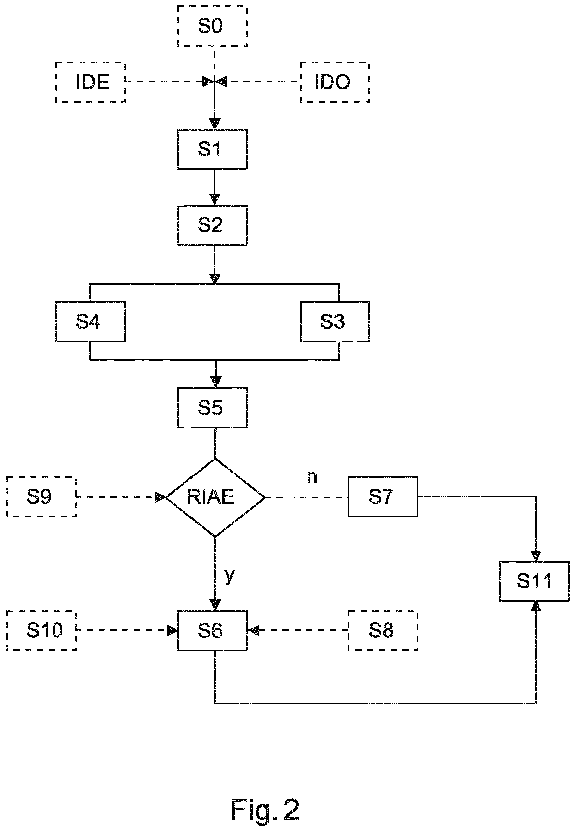

Fig. 2 shows a method for image acquisition according to the invention by use of the X-ray system 6 comprising an X-ray device 1.

In step 1 image acquisition is performed and an X-ray image is generated. The X-ray image is subject to automatic image analysis in step S2 and to an interpretation/analysis of the operator 3. At step S3 the operator 3 enters the operator decision of the X-ray image via the interface 9. The automatic image analysis yields an automatic analysis result at step S4. At step S5 the agreement level of operator decision and automatic analysis result are monitored. Remote image-analysis expertise RIAE is requested according to one of: agreement level, operator decision and/or automatic analysis result. If remote image-analysis expertise RIAE is necessary it is performed automatically by executing a decision escalation chain at step S6 to provide support for interpretation/analysis of the X-ray image, treatment and further steps. If no remote image-analysis expertise RIAE is necessary the agreement level is accordingly and a diagnosis, treatment or further action is provided at step S7.

In some embodiments, the decision escalation chain comprises selection of a best matching remote image-analysis expertise. In some embodiments, a brokering service provides remote image-analysis expertise (step S8).

In this embodiment, an operator interprets massive internal haemorrhage according to the X-ray image. The automatic analysis result however is different, so that the agreement level is rather low. Automatically remote image-analysis expertise is requested at step S6 by executing a decision escalation chain to provide selection of best matching remote image-analysis expertise. In this embodiment, the required expert could be a surgeon.

According to another embodiment, the decision escalation chain depends on diagnostic demands and/or requirements provided prior to image acquisition (step S0). Such demands could be according to the health-insurance of the patient which provides that only surgeons with a defined qualification are contacted for remote support. The decision escalation chain, will follow such demands and provide the respective surgeon. However, if the provided surgeon matches the health-insurance of the patient but is not the dedicated surgeon for the generated X-ray system, the decision escalation chain will provide the required expertise. In some embodiments, this can be automatic expertise if the confidence level of similar image interpretation/analysis is rated high, so that the operator can be supported. In this embodiment and also in embodiments, where a brokering system provides remote image-analysis expertise, the selection of the best matching expertise is performed automatically. However, according to the complexity of the case in some embodiments a manual selection can be performed as explained above.

The decision escalation chain also depends on the provided payment system. In some embodiments, the patient is identified IDO prior to image acquisition and respective information can be retrieved by the provided data. Alternatively, such information can be given to the operator for input to the X-ray system (S9), if remote image-analysis expertise RIAE is requested.

In some embodiments, such provisions depend on the operator expertise level which is identified according to the operator identification IDE. Accordingly, also option selectors 10 provided via the interface 9 are adapted to the operator expertise level. As default, basic level is provided and the option selector(s) and subsequent option selector(s) are provided in a step-by-step mode. The option selector(s) are adapted accordingly. If the operator is identified as expert, the option selector(s) and subsequent option selector(s) are adapted accordingly. If an expert is identified as operator, also the request for remote image-analysis expertise is adapted accordingly. In some embodiment, the expert operator can waive the request for remote image-analysis expertise RIAE. Also, an operator having according operator expertise level can overrule (S10) the decision escalation chain at each step. In any case the complete decision escalation chain, operator input and image data are documented and stored for follow-up analysis (S11).

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfil the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An X-ray device, comprising:
- an X-ray source, a detector,
- a processing unit comprising:
- image acquisition for generating an X-ray image;
- performing automatic image analysis of the X-ray image;
- comparing an automatic analysis result and an operator decision for the X-ray image and monitoring an agreement level of the comparison; and
- an interface for receiving the operator decision of the X-ray image and for requesting remote image-analysis expertise according to one of the agreement level, operator decision and/or automatic analysis result;
wherein requesting remote image-analysis expertise is performed automatically by executing a decision escalation chain.

2. An X-ray system, comprising:
- an X-ray device as in claim 1; and
- a connection to a remote expertise system;
wherein requesting remote image-analysis expertise is performed automatically by executing a decision escalation chain.

3. System according to claim 2, wherein the remote expertise system is a brokering system.

4. A method for image acquisition by use of an X-ray system, wherein the X-ray system comprises an X-ray device, the method comprising the following steps:
a) generating an X-ray image;
b) performing automatic image analysis of the X-ray image;
c) receiving an operator decision of the X-ray image;
d) monitoring an agreement level of an automatic analysis result and the operator decision;
e) requesting remote image-analysis expertise according to one of the agreement level, operator decision and/or automatic analysis result;
wherein the requesting remote image-analysis expertise is performed automatically by executing a decision escalation chain.

5. Method according to claim 4, wherein executing the decision escalation chain comprises selection of a best matching remote image-analysis expertise.

6. Method according to claim 4 or 5, wherein the method comprises determining diagnostic demands and/or requirements prior to image acquisition.

7. Method according to one of the claims 4 to 6, wherein the agreement level is classified as high if the automatic analysis result and the operator decision concur and requesting remote image-analysis expertise is waived.

8. Method according to claim 4, wherein a remote image-analysis expertise is requested for one of low level agreement, low quality operator decision or low quality automatic analysis result.

9. Method according to one of the claims 4 to 8, further comprising determining an operator expertise level.

10. Method according to claim 9, further comprising outputting at least one option selector according to the operator expertise level and/or object data.

11. Method according to claim 9, further comprising adapting a subsequent option selector according to the operator expertise and/or object data.

12. Method according to one of the claims 4 to 8, wherein the operator can waive the request for remote image-analysis expertise according to the operator expertise level.

13. Method according to one of the claims 4 to 13, wherein the decision escalation chain comprises an overruling function.

14. A computer program element for controlling an apparatus according to one of the claims 1 to 3, which, when being executed by a processing unit, is adapted to perform the method steps of one of the claims 4 to 13.

15. A computer readable medium having stored the program element of claim 14.
